# EUROPEAN PATENT APPLICATION

(11) **EP 1 114 620 A1**
(43) Date of publication of application: **11.07.2001**
(21) Application number: 00200056.0
(22) Date of filing: 07.01.2000
(51) Int. Cl.: A61B 18/20

(54) **Device for power treatment of tissue or biological samples with light**

(71) Applicant: Giga A/S, 2740 Skovlunde (DK)
(72) Inventor: Nörregaard, Jesper, 2610 Rödovre (DK); Lindvold, Lars, 2980 Kokkedal (DK); Bjerring, Peter, 8240 Risskov (DK)
(74) Representative: Elmeros, Claus

(57) **Abstract**

The invention relates to a laser device comprising at least one light source (LS), said device being adapted to establish a light wavelength at one of at least two possible wavelengths (PW), wherein at least two of the said possible wavelengths (PW) differ by less than 200 nm, less than 50nm or preferably less than 25nm.

According to the invention, even very narrow peaks corresponding to specific molecular structures may be excited by the same device. This feature of the invention implies that e.g. absorption peaks of water may be excited or, if necessary, avoided if no absorption of water is desired.

## Description

### Field of the invention

The invention relates to a device for power treatment or analysis of tissue or biological samples with light according to claim 1, a laser device comprising at least one light source according to claim 13, a laser treatment device for power treatment of skin with laser light according to claim 15, a method of power treatment or analysis of tissue or biological samples with light according to claim 21, a device for power treatment or analysis of tissue or biological samples with light according to claim 32 and a method of power treatment or analysis of tissue or biological samples with light according to claim 33.

### Background of the invention

To some extent, absorption of light in e.g. human tissue is well-described within the art, both with respect to analysis of the molecular structure of tissue by means of light and with respect to treatment of tissue as well.

Several examples of such techniques have been described within the art.

US 5,222,496 discloses a method of analysis, according to which e.g. blood may be examined non-invasively. The disclosed method utilizes the so-called NIR, near infrared spectrum, in order to obtain knowledge about e.g. the glucose concentration. Specifically, US 5,222,496 teaches a method of analysis implying at least two measurements of different wavelengths, i.e. a first data wavelength and a second reference wavelength.

A problem with the disclosed method is that other components than that to be analyzed may interfere with the obtained analysis result. Moreover, the obtainable signal-/noise ratio appears to be low.

US 5,527,350 discloses a method of treatment of psoriasis selectively with infrared radiation. The method implies selective "shooting" and destruction of certain blood vessels believed to be the carrier of psoriasis. The applied technology is based on AlGaAs semiconductor diodes having an approximate, maximum wavelength of 1000 nm.

A drawback with the method is that the obtained penetration depends heavily upon the individual characteristics of the skin to be treated.

### Summary of the invention

The invention relates to a device for power treatment or analysis of tissue or biological samples with light according to claim 1, said apparatus comprising at least one light source (LS), said device being adapted to establishing a light wavelength at one of at least two possible wavelengths (PW), each possible wavelength corresponding to the individual absorption properties of the power treated tissue or biological samples.

The power treatment may for instance advantageously be used for power treatment of live tissue, such as skin.

When, as stated in claim 2, at least two of the said possible wavelengths (PW) differ by less than 200 nm, less than 50nm or preferably less than 30nm, a further advantageous embodiment of the invention has been obtained.

When, as stated in claim 3, the said apparatus comprises at least two light sources (LS), a further advantageous embodiment of the invention has been obtained.

When, as stated in claim 4, at least two of the said possible light wavelengths may be established within a relatively narrow optical bandwidth, said optical bandwidth being full width of half maximum (FWHM), a further advantageous embodiment of the invention has been obtained.

When, as stated in claim 5, the said FWHM optical bandwidth of the at least two light wavelengths is less than 40nm, preferably less than 10nm, a further advantageous embodiment of the invention has been obtained.

When utilizing a narrow optical bandwidth, distinct narrow absorption structures may be excited for both treatment and analysis purposes.

When, as stated in claim 6, the possible wavelengths (PW) are established as a set of distinct predetermined wavelengths, a further advantageous embodiment of the invention has been obtained.

According to one embodiment of the invention, a power treatment device only features two predetermined wavelengths. Such a device may e.g. be operated as a traditional treatment device having an additional single switch may be changed if the operator wishes to alter the wavelength output of the laser treatment device. Of course, such a device may comprise both one single tunable laser light source, preferably a laser diode, or two light sources emitting laser light at two mutually different wavelengths.

Evidently, other embodiment according to the invention may constitute several laser light sources establishing light at several different wavelengths. Such a device may e.g. be an analysis apparatus addressing predefined molecular structures or bonds by a set of distinct possible wavelengths and/or by tuning/scanning over an interval of wavelengths of interest.

When, as stated in claim 7, at least one of the possible wavelengths (PW) is established by tuning of one of the light sources (LS), a further advantageous embodiment of the invention has been obtained.

When the possible wavelengths or at least some of them are set within a relatively narrow bandwidth, these wavelengths may be established by tuning of the light source(s).

It should be noted that tuning of the light source(s) is very advantageous when dealing with semiconductor laser light sources, i.e. laser diodes. Hence, laser diodes may be tuned into suitable wavelengths by means of relatively simple measures.

It should moreover be noted that the tuning of a light source, even within a narrow tunable band according to the invention, has turned out to be extremely advantageous due to the fact that even "minor" tuning of only a few nanometers may affect the absorption of tissue or biological samples considerably. Especially when using laser light of wavelengths in excess of 1100 nm.

Still, of course, tuning should allow for a band of wavelengths being broad enough for the desired purpose. Thus, if an absorption peak or curve covering a band of 30 nm is to be addressed, tuning should be within a corresponding range.

Moreover, the possible wavelengths should cover changes or variations of absorption properties due to e.g. temperature variations in the tissue or biological samples. Laser diodes feature a relatively broad tuning bandwidth combined with very high precision. Consequently, laser diodes represent a technically superior laser type and the laser diode is moreover very cost efficient.

When, as stated in claim 8, at least one of the laser sources comprises a laser diode, a further preferred embodiment of the invention has been obtained.

When, as stated in claim 9, the said diode is operating at wavelengths in excess of 1100nm. a further advantageous embodiment of the invention has been obtained.

When, as stated in claim 10m at least one of the said laser diodes is an InGaAsP diode, a further preferred embodiment of the invention has been obtained.

When, as stated in claim 11, the laser light from the laser source(s) (50) is delivered coincidentally from a beam delivery head (52), an advantageous embodiment of the invention has been obtained.

Hence, whether using the device manually or in a mechanically automated manner, the light may be delivered to the target from only one light emitting structure.

An example of a advantageous manually operated device of the above kind may be a skin treatment device enabling different penetration depths by one single beam delivery head. The operator will maintain the same feeling during operation independently of the applied laser beam.

A further example is a mechanically automated analysis system requiring high reproducibility. when delivering the beam from the same delivery head, no inaccuracy with respect to the exact spot of illumination will arise.

According to one preferred embodiment of the invention, an optical coupler may mix the light from the different applied light sources into one optical fiber forming the light emitting end of a beam delivery head or the pre-stage of a delivery head.

It should be noted that a coincident light delivery is of great importance when the scanning unit in the delivery head is the only one scanning unit needed for operation.

When, as stated in claim 12, the said device is adapted to establish a mean light wavelength at one of at least two possible mean wavelengths (PW) above 1100 nm, said possible mean wavelength (PW) having an spectral power density of at least 20 mW/nm, at least 50 mW/nm, or preferably 100 mW/nm, a further preferred embodiment of the invention has been obtained.

According to the invention, high power may be delivered within a narrow bandwitdh.

When, as stated in claim 13, a laser device comprises at least one light source (LS), said device being adapted to establish a light wavelength at one of at least two possible mean wavelengths (PW), wherein at least two of the said possible mean wavelengths (PM) differ by less than 200 nm, less than 50nm or preferably less than 25nm, a further advantageous embodiment of the invention has been obtained .

Preferably, at least one of the possible wavelengths is provided by means of a laser light diode.

When, as stated in claim 13 the said device is adapted to establish a mean light wavelength at one of at least two possible mean wavelengths (PW) above 1100 nm, said possible mean wavelength (PW) having an spectral power density of at least 20 mW/nm, preferably at least 50 mW/nm, a further advantageous embodiment of the invention has been obtained.

When, as stated in claim 15, a laser treatment device for power treatment of skin with laser light comprises at least one laser light source () having wavelengths longer than 1100 nm, a further advantageous embodiment has been obtained .

Laser treatment devices having wavelengths in excess of 1100 nm benefit from the fact that more specific excitations may be obtained more or less independently of the skin type.

According to the invention, even narrow peaks corresponding to particular molecular structures may be excited. This feature of the invention implies that e.g. absorption peaks of water may be excited or, if necessary, avoided if no absorption of water is desired.

A further important feature of the invention is that treatment of e.g. human skin may be facilitated more or less independently of the skin type due to the fact that the broadband excitation of melanin at wavelengths of less than 1100 nm is considerably smaller and more concentrated at longer wavelengths.

An important feature of the invention is that illumination at wavelengths in excess of approximately 1100 nm, i.e. in the so-called low-order overtone region, may excite more specific molecular structures due to the fact that low-order overtones or fundamental tones gradually dominate e.g. human tissue or biological samples as the wavelength increases. Hence, at approximately 1400 nm, first-order resonance may be observed.

When, as stated in claim 18, the said at least one laser light source comprises means for tuning the wavelength of the source within a tunable range (TR) in excess of 10nm, a possibility of tuning the wavelength relative to a wavelength already applied has been obtained.

Moreover, the invention relates to a method of power treatment of tissue or biological samples with light as stated in claim 23, wherein the wavelength or at least one of the wavelengths of the said light is established at one of several possible wavelengths (PW), each possible wavelength corresponds to the individual absorption properties of the treated tissue or biological samples.

When choosing different wavelengths from a selection of possible wavelengths according to desired absorption properties, the possibility of selective treatment has been obtained. Thus, chosen absorption peaks or other applicable absorption structures of the tissue or biological samples may be chosen and selectively addressed.

Moreover, the invention relates to a device for power treatment or analysis of tissue or biological samples with light as stated in claim 32, said apparatus comprising at least one light source (LS), said device being adapted to establish at least one possible mean light wavelength (PW) above 1100 nm, said possible mean light wavelength (PW) having a spectral power density of at least 20 mW/nm, preferably at least 50 mW/nm.

According to the invention, the delivery of a relatively high power at a narrow wavelength range in order to obtain a specific excitation of very narrow absorption peaks or structures has become possible with the invention.

### Brief description of the drawings

The invention will be described below with reference to the drawings where
- fig.1: shows the mechanical design of the diode and the corresponding diode sub-carrier according to the invention,
- fig.2: shows the LIV characteristics of an applied laser diode
- fig.3: illustrates the spectra of a laser diode at different temperatures of the diode sub-carrier,
- fig.4: illustrates schematically the basic components of an illumination device of the invention,
- figs.,5A-C: illustrate power treatment of human tissue
- fig.6: illustrates the tissue temperature rise vs. wavelengths of the laser diode,
- fig. 7: illustrates the absorption from 1100nm to 2500 nm of molecular bonds of biological samples
- fig.8: illustrates components applicable to the embodiment of fig. 4,
- fig.9: illustrates schematically a treatment or analysis device according to the invention, and
- fig. 10-12: illustrates schematically additional treatment or analysis devices according to the invention,

### Detailed description

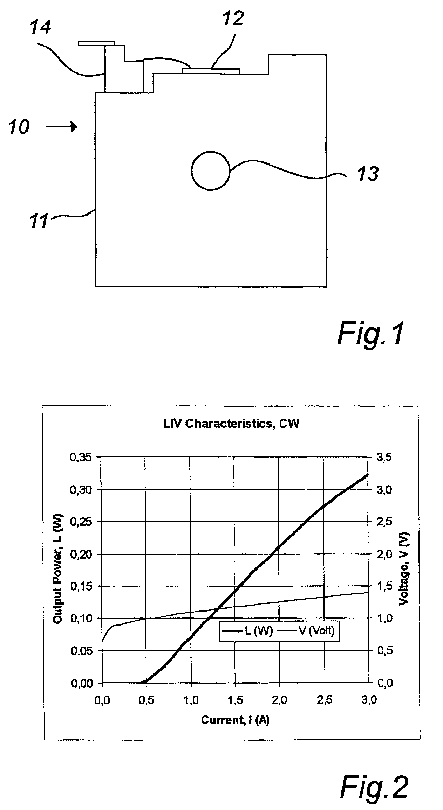

Fig. 1 shows the mechanical design of the diode and the corresponding diode sub-carrier utilized according to one preferred embodiment of the invention. Initially, it should be emphasized that the described embodiments and the utilized components constitute possible options within the scope of the invention which are available to a man skilled within the art.

The applied laser diode according to the below-described preferred embodiments of the invention is as commercially available laser diode manufactured by the applicant.

The laser diode is a GD20482 high power Fabry-Perot laser diode designed as a source with a high brightness mounted on an open heat sink (un-encapsulated).

Fig 1 shows the applied laser device 10 comprising a gold-plated copper-tungsten stub 11. A laser die 12 is soldered to the stub. A stub may e.g. also be referred to as a sub-carrier.

The laser 12 is wire-bonded to a gold plated ceramic block 14 forming a cathode connection.

The stub is used for an anode connection of the laser die 12.

The stub 11 , i.e. the heat sink, may be attached to a cooling device (not shown) by means of a mounting hole 13 in the stub.

Source mean wavelengths at room temperature are ranging from 1400 to 1550 nm depending on the desired application, but the source is designed in such a manner that a tuning of +/- 30 nm is possible by a change in the heat sink temperature.

The laser die is flip-chip mounted on an open heat sink with low thermal resistance as result. The high efficiency of the multiple quantum well structure provides a low junction temperature and extended lifetime as well as increased reliability. The open heat sink must be attached to a larger heat sink providing dissipation of thermal energy.

The laser diode is a multiple quantum well laser with InGaAsP epilayers grown on InP. The source is a partly coherent gain-guided broad area emitter with closely spaced stripes. This results in a more uniform emission across the emitting aperture. The aperture is approximately 100 microns wide. The laser operates in multiple longitudinal modes. The radiation is nearly Gaussian in the plane perpendicular to the junction. In the plane parallel to the laser diode junction, the radiation exhibits a complex pattern typical for a broad area emitter.

Evidently, other types of laser sources or laser diodes are applicable according to the invention. Nevertheless, the GD20482 represents a very attractive laser source in the sense that an InGaAsP laser diode generally generates wavelengths that may be advantageously utilized by the invention. This feature will be dealt with in detail below.

Fig.2 shows the light current voltage characteristics (LIV) of the applied laser diode, continuous wave (CW).

The light output power L is illustrated by the full line. The voltage drop over the laser V is illustrated by the dotted line.

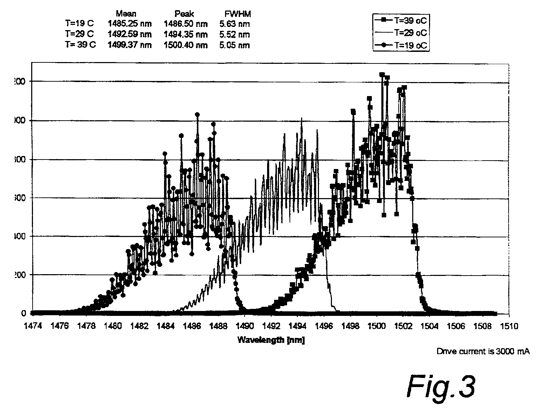

Fig.3 illustrates the spectra of the above-mentioned laser diode at different temperatures.

It appears that the mean wavelength may be adjusted quite accurately by regulation of the temperature of the laser diode. The present diode has been temperature-regulated by means of a commercially available Peltier element.
Evidently, other adjustments than the above-mentioned temperature regulation may be made.

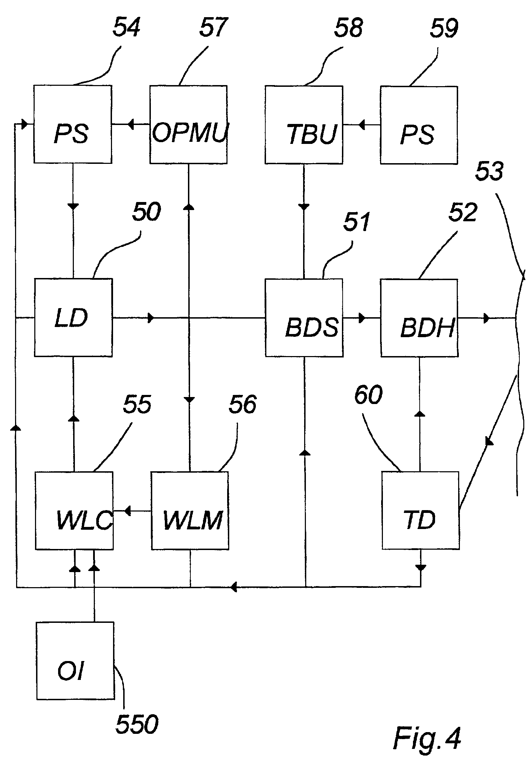

Fig 4 illustrates the basic components of an illumination device according to the invention. The device is intended for illumination of tissue or biological samples. Specifically, the illustrated device is intended for power treatment or analysis of tissue or biological samples.

The device comprises a laser device 50 optically connected to a beam delivery system 51, which is, in turn, optically connected to a beam delivery head 52.

As an example, the laser device comprises a high power InGaAsP laser diode described in the above-mentioned figs. 1-3. The laser diode emits a room temperature mean wavelength, XRT within the range of 1100 nm to 1600 nm. The room temperature mean wavelenght λ_{RT} may also be chosen within other ranges from 1100 nm to 4000 nm.

Other types of laser diodes may be used for longer wavelengths of up to approximately 4000 nm such as other group III-V semiconductor laser diodes or group II-VI semiconductor laser diodes.

The laser device is powered by a power supply 54 and controlled by a wavelength controller 55.

An optical power measuring unit 57 measures the output of the laser device 50 and feeds a corresponding signal back to the power supply 54.

Likewise, an optical wavelength measurement unit 56 measures the wavelength of the laser device 50 and feeds a corresponding signal back to the wavelength controller 55.

Moreover, the beam delivery system 51 comprises a tracking beam 58 powered by a corresponding power supply 59. The primary function of the established tracking beam unit 58 is to establish a visible aiming beam to be delivered by the beam delivery head 52 towards the target 53 to be illuminated. This feature helps the operator take proper aim with the invisible optical laser beam delivered by the laser device 50.

By the beam delivery head, a spatial scanning of the laser beam across the target, e.g. the human skin, may take place. In addition to this spatial scanning (or without the spatial scanning), a wavelength scanning can be performed using this piece of equipment. In some cases, e.g. for human skin, the wavelength scanning leads to a scan of the magnitude of the penetration of the skin. The wavelength scanning may not be continuous but can be performed very fast (kHz range) over the entire wavelength range using, e.g., the multiple laser device configuration. The multiple laser device configuration is described below.

A further component is a target diagnostics unit 60 which is described below.

The last component is an operator interface 550 communicating with the wavelength controller 55. An operator interface according to the present embodiment is applied as a simple switch (not shown) which may be operated to provide one of a number of predefined wavelengths settings.

It should be noted that several other types of operator interfaces may be applicable within the scope of the invention, such as step-less tuning, establishing of beams combined of several different wavelengths.

One of the core components of the illustrated device is the wavelength controller 55 enabling the user to easily establish a certain desired wavelength within a tunable range at a given accuracy. One of several applicable regulations may be performed by temperature control of the laser by means of a Peltier element.

A Peltier element, also mentioned within the art as a thermoelectric cooler, is commercially available from e.g. Marlow Industries Inc.

According to the present illustrated embodiment, a change in temperature of +/-1 °C results in a corresponding change of approximately +/- 0.7nm.

According to the present embodiment, the tuning period will last approximately one minute per five degrees.

The illustrated device may be tuned by the operator within a range of XRT +/- 30 nm, where λ_{RT}, may be established within the range of [1100nm; 1600nm], λ_{RT} being the room temperature mean wavelength of the diode.

Below, the above-mentioned basic components will be dealt with in detail.

The described laser device(s) 50 may for instance be a solid state laser, a gas laser, a dye laser, a polymer based laser, a fiber laser, a broad area laser diode, a laser diode array, a laser diode bar consisting of multiple broad-area lasers or diode arrays, a flared laser diode device, a ridge waveguide laser diode, a buried heterostructure laser diode, a tapped laser diode device, a monolithically or hybrid integrated low power device with a gain section for amplification of the low-power radiation. The gain section may either be designed for single pass, double pass or multi-pass amplification, and may therefore also be a laser geometric itself. The gain section may either be electrically or optically pumped.

A low-power device may be a DFB-, a DBR-,a Fabry-Perot laser, or a VCSEL based on semiconductors (either spatial single mode or multi-mode), a fiber laser (DFB or DBR or Fabry-Perot).

A gain section may be any of the initially mentioned laser devices, a doped solid state medium or a doped optical fiber.

Semiconductor devices may have a bulk, a single-, a multi-quantum well, or a quantum dot structure. In addition, semiconductor devices may consist of single or multiple electrical sections. Furthermore, the optical confinement layout may contain tappered sections for mode-sharping.

The power supply 54 may run the laser device in any of the three modes: continuous wave, quasi-continuous wave or pulsed.

The wavelength controller 55 may include a control circuit and driver to control the wavelength of the laser device. If the wavelength is controlled by adjusting the temperature of the laser device, the driver may be connected to, e.g., a Peltier element. If the wavelength is controlled by an adjusting tilt of a grating, the driver may be connected to e.g. a piezo element which is connected to the grating.

The wavelength measurement unit 56 may use e.g. a spectrometer, detection of the electrical beat of a photo detector between the light from the laser device and a reference light beam, the detector of the power being transmitted through a specially designed multi-layer filter with an edge-profile (either a high-pass or low-pass optical filter), a scanning Fabry-perot etalon, or a Michelson interferometer.

Furthermore, the desired wavelength of the laser for a given treatment or analysis may be established through the following method: a fraction of the light is sent trough a calibration material and the transmitted power is measured. The calibration material possesses tailored absorption or transmission properties, e.g., a single absorption peak within a certain wavelength of interest. This absorption peak is centered at a known and prefixed wavelength. The wavelength of the laser device may then be determined by continuous tuning of the laser wavelength, e.g., by changing the temperature of the laser device until the measured power has reached a minimum or maximum in cases where the tailored properties represent a single transmission line. If the desired wavelength differs from the prefixed wavelength, the tuning of the laser device can then be performed with knowledge of the (tuning) settings resulting in a laser wavelength equal to the prefixed wavelength. The calibration material may for example be a tailored organic solution that may be liquid or solid, an organic or non-organic material incl. polymers, living material (hosted in liquid or solid-like material). The calibration material may also be a filter such as an interference multi-layer filter, prism based device or Fabry-Perot etalon.

The target diagnostician 60 may measure a number of characteristics of the light-target interaction. The measurement may include thermal dissipation, beam size, target temperature, spatial temperature profile, penetration depth, power, absorbed power, reflected power, luminescence, wavelength, fluorescence and the light scattering profile. The measurement can be used to control the wavelength or adjust the drive current of the laser device. Furthermore, the measurement can be used to control the beam delivery system and the head by e.g. adjustments of the spatial scan geometry or beam focus etc.

The optical power measuring unit 57 provides information about power emitted from the laser device, and eventually delivered at the target. This information may be obtained by using a photo detector that measures a small fraction of the total power. The small fraction may be obtained by using e.g. a beam-splitter or another reflected part of an optical component. Furthermore, the small fraction may be the power emitted from the back facet of the laser device.

The beam delivery system 51 may be either free-spaced or based on optical fibers. The system may include optical components, such as lenses and beam splitters, for beam shaping and/or coupling of the light from the laser device to an optical fiber, and coupling to the laser delivery head. Moreover, the delivery system may include arrangements for spatial overlapping of the tracking beam with the beam from the laser device.

The tracking beam unit 58, i.e. a beam that indicates the propagation or the focal point of the beam of the laser device with a wavelength in the visible region, e.g. a red laser diode, may be added to the system in order to ease the pointing of the beam from the laser device towards the target.

The delivery head 52, which may be hand-held or stationary, may include adjustable optical components for the shaping of the beam obtained from the beam delivery system, adjustable mirrors for implementation of the spatial scanning, shutters for turn-off of the laser beam. Moreover, the delivery head may include arrangements for spatial overlapping of the tracking beam with the beam from the laser device.

With respect to control of the wavelength according to the invention, several methods may be applicable.

The laser wavelength of the laser device may for example be controlled by using:
1) Active heating or cooling of the laser device, thereby adjusting the temperature of the laser device.
2) Adjustment of the applied drive current for the laser device.
3) External cavity formed between a reflector, e.g. an ordinary mirror or a phase conjugating mirror, and the laser device with a frequency selective element in the external cavity. The frequency selective element may be a Fabry-Perot etalon, a multi-layer filter such as an interference filter, a diffraction grating, a holographic grating element, a dispersive element such as a prism.
4) External feedback (formation of an external cavity) from a diffraction grating or a more complex holographic grating element arranged in e.g. a Littrow configuration or a Grazing incidence configuration. The laser wavelength is determined by the tilt of the grating or the tilt of a mirror in case of a folded cavity. The external feedback may be applied to either the front facet or the rear facet of the laser device.
5) A monolithically or hybrid integrated DBR (distributed Bragg-Reflector) section controlled by its own electrical contact. The laser wavelength is determined by the effective index of refraction, which is influenced by the amount of electrical current fed through the DBR section.
6) A External cavity formed between the laser device and a Bragg grating in an optical fiber connected to the beam delivery system. The laser wavelength is controlled by altering the period or the effective index of refraction of the Bragg grating using e.g. thermal heating of the grating or mechanically induced stress such as pulling, compression or bending. The mechanical stress may be applied by e.g. attachment of the fiber with the Bragg grating to a piezo electrical element.
7) Injection locking of the high power laser device using an external laser such as a low power DFB-, DBR- or Fabry-Perot laser. The wavelength of the laser device is controlled by controlling the laser wavelength of the low power laser using any of the above mentioned methods. The injection locking can be applied to the laser device with either an on-axis or an off-axis configuration.
8) Using the laser device as a gain section (single pass, double pass or multi-pass configuration) in which the radiation from an external laser such as a low-power laser is amplified. The gain section and the low-power device may be monolithically integrated. The wavelength of the laser device is controlled by controlling the laser wavelength of the low-power laser using any of the above mentioned methods.
9) Multiple laser device configuration. The wavelength at the target may also be controlled by the use of more than one laser device; the different laser devices all have prefixed but different wavelengths. The output power from the different laser devices is combined at the same spatial position, e.g. in the same optical fiber. Turning on the laser device with the desired wavelength subsequently controls the wavelength at the target. In addition, the prefixed wavelength for the individual lasers may be controlled or set by using any of the above-mentioned techniques.

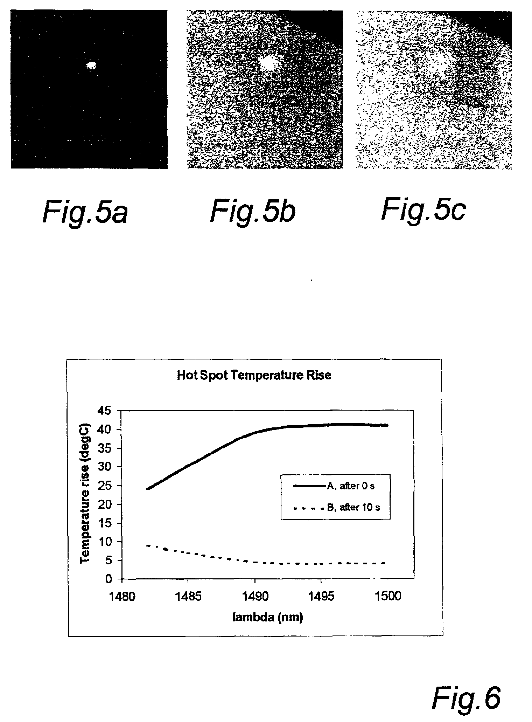

Figs. 5A-C illustrate one of several applications of the laser light. According to the illustrated experiment, a laser device of the above-mentioned type has been utilized for radiation of a birthmark on an arm.

The birthmark is illuminated by a laser light having a mean wavelength of 1492 nm, an optical full width of half maximum bandwidth, FWHM, of approximately 6 nm, and optical power of 0.22 W.

Fig. 5A shows an infrared, IR, thermography of the arm during illumination of the birthmark.

The temperature of the illuminated spot, i.e. the small white area, is approximately 70 °C.

The high temperature has been obtained by excitation of the molecular structures of the skin by means of the laser light.

Fig. 5B shows an IR thermography of the arm 10 seconds after ending the illumination process.

Gradually, the temperature will now drop due to the cooling from the ambient air and tissue. Moreover, the blood flow will contribute to the cooling process.

Fig. 5C shows an IR thermography of the arm 30 seconds after ending the illumination process.

The temperature of the illuminated spot is now quite close to the temperature of the surrounding tissue, and the birthmark has been damaged.

Subsequently, new tissue will replace the damaged tissue.

The above-mentioned removal of a birthmark has several interesting aspects which will be described in detail at a later point.

First of all, it should be noted that high-power treatment may now be used above wavelengths of 1100nm.

Secondly, some kind of shallow penetration has been obtained due to the fact that the elevated temperature is sustained for a short time after ending the illumination process.

In order to clarify and explain these aspects in detail, the experiment has been illustrated and established and will be described below.

In order to visualize further interesting details of the absorption in human skin, a tomato has been experimentally radiated by the above-mentioned laser light device of fig. 4. Of course, it should be emphasized that the tomato hardly represents e.g. human tissue. Obviously, a tomato lacks the properties of live tissue or certain biological samples. Nevertheless, the structure of the surface of a tomato has certain physical similarities to human skin.

The present experiment serves for illustrative purposes only and should in no way narrow the scope of the invention.

The temperatures have been measured by IR thermography.

Initially, a beam delivery head radiated a tomato. The initial mean radiation wavelength was approximately 1500 nm and the optical power approximately 0.22W. The temperature was measured immediately after ending the radiation process, and the peak temperature at the center of the radiated sample was approximately 42 °C higher than the surrounding surface of the non-illuminated part of the tomato.

Ten seconds after ending the radiation process, the temperature of the hot spot was measured again. At this point, the temperature was approximately 4 °C higher than the surrounding surface.

The second time, a beam delivery head radiated a tomato. The mean radiation wavelength was now tuned to be approximately 1493 nm and the optical power approximately 0.22W. The temperature was measured immediately after ending the radiation process, and the peak temperature at the center of the radiated sample was approximately 40 °C higher than the surrounding surface of the non-illuminated part of the tomato.

Ten seconds after termination of the radiation, the temperature of the hot spot was measured again. At this point, the temperature was approximately 4-4.5°C higher than the surrounding surface.

The third time, a beam delivery head radiated a tomato. The mean radiation wavelength was now tuned to be approximately 1485 nm and the optical power approximately 0.22W. The temperature was measured immediately after ending the radiation process, and the peak temperature at the center of the radiated sample was approximately 30 °C higher than the surrounding surface of the non-illuminated part of the tomato.

Ten seconds after termination of the radiation, the temperature of the hot spot was measured again. At this point, the temperature was approximately 7 °C higher than the surrounding surface.

The obtained results have been illustrated in fig. 6 showing a rough presentation of the obtained temperatures at different wavelengths of the laser diode of fig. 3 according to the above-mentioned experiments.

Initially, it should be noted that the three different wavelengths feature quite different absorption characteristics notwithstanding the fact that the applied wavelengths are mutually very close.

The smaller hot spot temperature rise at 1485 nm cannot be explained by assuming that the applied power at 1485 nm was less than the applied power at 1493 nm and 1500 nm as the temperature rise in the hot spot illuminated by a 1485 nm mean wavelength ten seconds after termination of radiation was higher than the corresponding temperature rises in the hot spots illuminated by 1493 nm and 1500 nm wavelengths.

Hence, referring now to the roughly illustrated curve A, the temperature differences between the hot spot surface temperatures and the surrounding non-illuminated surfaces measured immediately after ending the radiation process indicate that the obtained absorption by the sample depends heavily on the applied wavelength.

Moreover, curve B representing the measured temperature differences ten seconds after ending the radiation process of the sample, indicates that the penetration depth depends on wavelength as well even though the curve is not identical with curve A.

The test results suggest that the penetration depth depends heavily upon the excitation wavelength. Hence, according to the invention, individual types of molecular bonds may be individually treated by means of power. Moreover, the invention benefits from the fact that individual absorption peaks corresponding to different types of bonds become separate and quite distinct as the wavelength increases due to the fact that the present excitations gradually become low-order overtone excitations. This feature will be described in detail below.

Moreover, the curves show that power treatment of tissue or biological samples may be controlled much more selectively than is possible with prior art.

It should be noted that the curves illustrate that the possibility of a specific excitation at certain wavelengths may be obtained with laser light having mean wavelengths comparable to the absorption wavelengths of the sample/tissue to be radiated.

A further and very important feature of a radiation device according to the invention is that a device having only one or a few laser diodes may feature several different absorption properties. This provision is not only interesting because of the financial considerations, but it is also important when dealing with a power treatment device intended for medical use as a device having a simple operational interface is highly appreciated by medical operators.

A further important aspect of the invention is that even high selective absorption properties may be obtained from wavelengths as short as approximately 1100nm, and that these different absorption properties may be obtained by means of very small variations in the exciting wavelengths of the applied laser beams. This feature is extremely valuable when dealing with laser diodes, which may be tuned dynamically and in such a way that one laser diode may feature several different absorption peaks or flanges even within a quite narrow tunable range of e.g. 20-40nm.

The absorption and penetration properties are changed by tuning of the laser diode wavelength. The wavelength is for example tuned to other absorption and penetrations depths by changing the laser diode sub-carrier temperature a few degrees Celcius.

It should be noted that the presence of distinct addressable absorption peaks increases when the wavelength increases. This feature, combined with a laser diode's ability of high power optical emission within a narrow optical band, provides an advantageous method of analysis or power treatment of samples with laser light having several closely arranged wavelengths. As mentioned above, these several wavelengths may be applied by several laser diode sources and/or tunable laser diodes.

A further aspects of the invention is that the laser light wavelengths provided by a laser diode or several laser diodes may be established without complicated optical filter structures such as those applied in systems having broadband sources.

A further aspect of the invention is that laser treatment devices having wavelengths longer than 1100 nm benefit from the fact that more specific excitations may be obtained more or less independently of the skin type. According to the invention, even narrow peaks corresponding to specific molecular structures may be excited.

This feature of the invention implies that e.g. absorption peaks of water may be excited or, if necessary, avoided if no absorption of water is desired.

A further important feature of the invention is that treatment or analysis of e.g. human skin may be facilitated more or less independently of the skin type due to the fact that the absorption is substantially independent of the skin color or pigmentation above 1100 nm. Hence, power treatment or analysis of skin tissue below 1100 nm tends to be masked by especially excitation of melanin.

A further important feature of the invention is that illumination at wavelengths longer than approximately 1100 nm, i.e. in the so-called overtone region, may excite more specific molecular structures due to the fact that low-order overtones gradually dominate e.g. human tissue or biological samples as the wavelength increases. Hence, at approximately 1400 nm, first-order resonance may be observed.

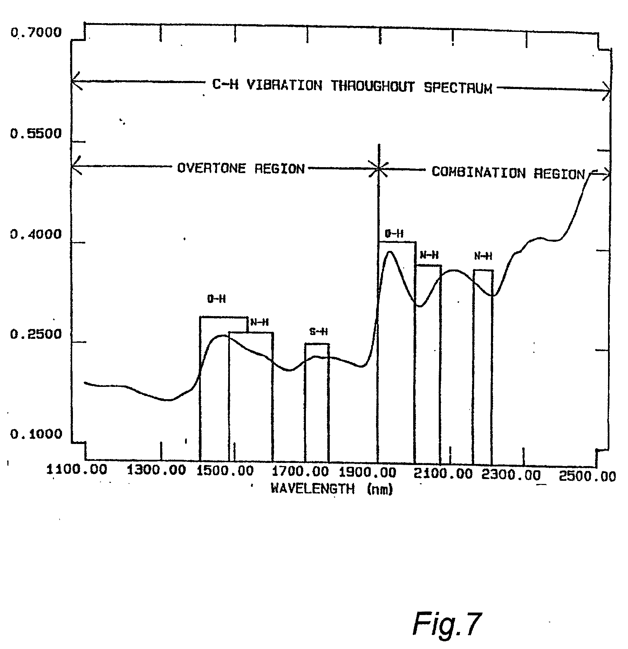

- Fig. 7: illustrates the absorption of molecular bonds of biological samples within a wavelength range of [1100nm; 2500nm].

It appears that the somewhat diffuse presence of high order resonance of tissue bonds becomes slightly more distinct in the so-called overtone region in which first-order resonance may be found. When entering the combination region, zero-order resonance, i.e. the fundamental resonance, may be obtained.

As mentioned above, the invention benefits from the fact that certain laser diode properties match biological properties due to the fact that the applied laser diodes may address the present narrow peaks and structures of the overtone region selectively, i.e. wavelengths longer than 1100nm. Moreover, the laser diodes may be tuned dynamically to cover not only one static wavelength, but a range of wavelengths.

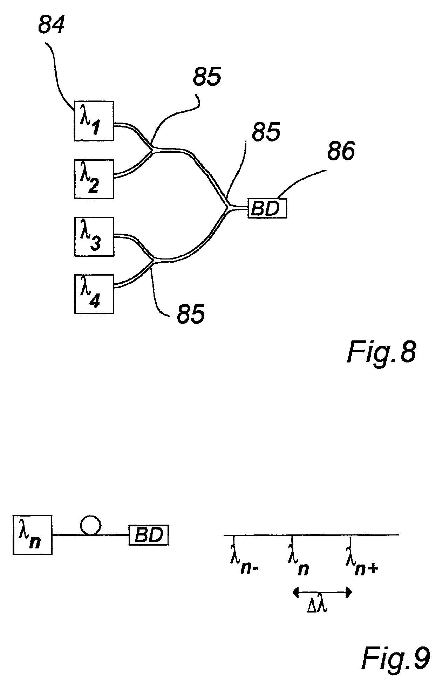

Fig. 8 illustrates one of several possible ways of coupling light from different light sources 84 into one beam delivery head 86 via optical couplers 85.

Evidently, several application within the fields of treatment or analysis will benefit from a coincident delivery to the beam delivery head 86. Especially when dealing with more complicated designs of the delivery head 86 as the above-mentioned spatial scanning types.

Fig. 9 illustrates a preferred embodiment of the invention in which a tunable laser light source of the above-mentioned type emits light to a beam delivery head BD via optical fiber means.

According to the below-mentioned embodiment, the laser devices are adapted to establish a mean light wavelengths having an spectral power density of at least 20 mW/nm, at least 50 mW/nm, or preferably 100 mW/nm. Hence, according to the invention, high power may be delivered within a narrow bandwidth.

The laser light sources emits laser light at a wavelength λn at room temperature and may be tuned, e.g. by a change in temperature of the laser diode within a range of λn- and λn+.

The disclosed device may e.g. by applicable to power treatment of human skin or for analysis of biological samples.

When dealing with a medical application operated by medical personnel, simple operator interface is preferred such as a simple switch providing e.g. only two or three predefined different wavelengths, such as λn , λn- and λn+.

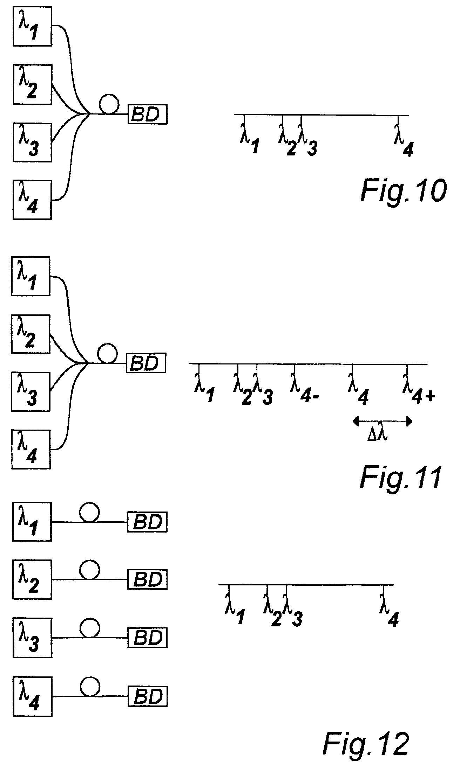

Fig. 10 illustrates a further preferred embodiment of the invention in which four laser light sources, preferably semiconductor laser light sources, emit light to a beam delivery head BD via optical fiber means. The four different light sources may be switch on and off sequentially or simultaneously if desired. The four possible wavelengths λ1 , λ2, λ3 and λ4 are distinct and non-adjustable by the operator, but may naturally still be provided at the beam delivery head individually or in combination as desired by the operator.

The illustrated device may e.g. be pre-designed for analysis of blood with respect to glucose or other relevant molecular structures.

The illustrated device may further be pre-designed for power treatment of skin at four different wavelengths λ1, λ2, λ3 and λ4. The predefined, non-adjustable wavelenghts controlled by the operator provide four different penetration depths and are therefore carefully adjusted to the absorption peaks of skin with wavelengths longer than 1100nm.

Fig. 11 illustrates a further preferred embodiment of the invention in which four laser light sources, preferably semiconductor laser light sources, emit light to a beam delivery head BD via optical fiber means. The four different light sources may be switch on and off sequentially or simultaneously if desired. The three possible wavelengths λ1, λ2 and λ3 are distinct and non-adjustable by the operator, but may still, of course be provided at the beam delivery head individually or in combination as desired by the operator. The fourth possible wavelength λ4 may be chosen by the operator by means of the operator interface (not shown), and range from λ4- to λ4+.

The illustrated device may e.g. be pre-designed for analysis of blood with respect to glucose or other relevant molecular structures, still providing the operator with the possibility of monitoring the wavelength range from λ4- to λ4+ more carefully.

Obviously, further configurations of the devices may be applicable within the scope of the invention, such as a multi-light source device in which all the laser light sources may be tuned manually or automatically. Hence, very detailed scannings or sweeps may be applied according to the invention.

Fig. 12 illustrates a further preferred embodiment of the invention very similar to the device of fig. 10 in which four laser light sources, preferably semiconductor laser light sources, each emit light to a corresponding beam delivery head BD via optical fiber means. The four different light sources may be switched on and off sequentially. The four possible wavelengths λ1, λ2, λ3 and λ4 are distinct and non-adjustable by the operator but may of course be selected by the operator.

## Claims

1. Device for power treatment or analysis of tissue or biological samples with light, said apparatus comprising at least one light source (LS), said device being adapted to establish a light wavelength at one of at least two possible wavelengths (PW), each possible wavelength corresponding to the individual absorption properties of the power treated tissue or biological samples.

2. Device for power treatment or analysis of tissue or biological samples with light according to claim 1, wherein at least two of the said possible wavelengths (PW) differ by less than 200 nm, less than 50nm or preferably less than 30nm.

3. Device for power treatment or analysis of tissue or biological samples with light according to claim 1 or 2, wherein said apparatus comprises at least two light sources (LS).

4. Device for power treatment or analysis of tissue or biological samples with light according to claims 1-3, wherein at least two of the said possible light wavelengths may be established within a relatively narrow optical bandwidth, said optical bandwidth being full width of half maximum (FWHM).

5. Device for power treatment or analysis of tissue or biological samples with light according to claims 1-4, wherein the said FWHM optical bandwidth of the at least two light wavelengths is less than 40nm, preferably less than 10nm

6. Device for power treatment or analysis of tissue or biological samples with light according to claims 1-5, wherein the possible wavelengths (PW) are established as a set of distinct predetermined wavelengths.

7. Device for power treatment or analysis of tissue or biological samples with light according to claims 1-6, wherein at least one of the possible wavelengths (PW) is established by tuning of one of the light sources (LS).

8. Device for power treatment or analysis of tissue or biological samples with light according to claims 1-7, wherein at least one of the laser sources comprises a laser diode.

9. Device for power treatment or analysis of tissue or biological samples with light according to claim 8, wherein the said diode is operating at wavelengths longer than 1100nm.

10. Device for power treatment or analysis of tissue or biological samples with light according to claim 9, wherein at least one of the said laser diodes is an InGaAsP diode.

11. Device for power treatment or analysis of tissue or biological samples with light according to claims 1-10, wherein the laser light from the laser source(s) (50) is delivered coincidentally from a beam delivery head (52).

12. Device for power treatment or analysis of tissue or biological samples with light according to claims 1-11, wherein said device is adapted to establish a mean light wavelength at one of at least two possible mean wavelengths (PW) above 1100 nm, said possible mean wavelength (PW) having a spectral power density of at least 20 mW/nm, preferably at least 50 mW/nm.

13. Laser device comprising at least one light source (LS), said device being adapted to establish a light wavelength at one of at least two possible mean wavelengths (PW), wherein at least two of the said possible mean wavelengths (PM) differ by less than 200 nm, less than 50nm or preferably less than 25nm.

14. Laser device for power treatment or analysis of tissue or biological samples with light according to claim 13, wherein said device is adapted to establish a mean light wavelength at one of at least two possible mean wavelengths (PW) above 1100 nm, said possible mean wavelength (PW) having an spectral power density of at least 20 mW/nm, preferably at least 50 mW/nm.

15. Laser treatment device for power treatment of skin with laser light, said device comprising at least one laser light source () having wavelengths longer than 1100 nm.

16. Laser treatment device according to claim 15, wherein the said laser light source comprises at least one laser diode ().

17. Laser treatment device according to claim 15 or 16, wherein the said at least one laser light source () has a wavelength of less than 4000 nm.

18. Laser treatment device according to claims 15-17, wherein the said at least one laser light source comprises means for tuning the wavelength of the source within a tunable range (TR) in excess of 10nm.

19. Laser treatment device according to claims 15-18, wherein said tunable range is in excess of 10 nm.

20. Laser treatment device according to claims 15-19, wherein said at least one laser light source () emits light at an optical bandwidth of less than 50nm, preferably less than 10nm, said optical bandwidth being FWHM.

21. Method of power treatment or analysis of tissue or biological samples with light from at least one laser diode light source (), said light being substantially monochromatic, the wavelength or at least one of the laser diode sources of the said light being established at a first wavelength to correspond to the desired absorption properties of the skin/tissue or biological samples,
said method also comprising tuning of at least one laser diode light source to a wavelength differing from the said at least first wavelength in such a way that the said absorption properties change.

22. Method of power treatment or analysis of tissue or biological samples with light from at least one laser diode light source (), said light being substantially monochromatic, the wavelength or at least one of the laser diode sources of the said light being established at a first wavelength to correspond to certain desired absorption properties of the skin/tissue or biological samples,
said method also comprising tuning of at least one laser diode light source to a wavelength differing from the said at least first wavelength.

23. Method of power treatment of tissue or biological samples with light, wherein the wavelength or at least one of the wavelengths of the said light is established at one of several possible wavelengths (PW), each possible wavelength corresponding to the individual absorption properties of the treated tissue or biological samples.

24. Method of power treatment of tissue or biological samples with light according to claim 23, wherein at least two of the said possible wavelengths (PW) differ by less than 200 nm, less than 50nm or preferably less than 30nm.

25. Method of power treatment of tissue or biological samples according to claim 23 or 24, wherein each of at least two of the said light wavelengths are established within a relatively narrow optical bandwidth, said optical bandwidth being full width of half maximum (FWHM).

26. Method of power treatment of tissue or biological samples according to claims 23-25, wherein said optical bandwidth of each of the at least two light wavelengths is less than 40nm, preferably less than 10nm.

27. Method of power treatment of tissue or biological samples according to claims 23-26, wherein the said light is a laser light.

28. Method of power treatment of tissue according to claims 23-27, wherein the said wavelengths are established close to the resonance peaks of the tissue or biological samples to be treated.

29. Method of power treatment of tissue according to claims 23-28, wherein the said tissue or biological samples is comprised by human skin.

30. Method of power treatment of tissue or biological samples according to claims 23-29, wherein the said certain predetermined absorption properties are determined by the penetration depth.

31. Method of power treatment of tissue or biological samples according to claims 23-30, wherein the mean light wavelengths have a spectral power density of at least 20 mW/nm, preferably at least 50 mW/nm.

32. Device for power treatment or analysis of tissue or biological samples with light, said apparatus comprising at least one light source (LS), said device being adapted to establish one possible mean light wavelength (PW) longer than 1100 nm, said possible mean light wavelength (PW) having a spectral power density of at least 20 mW/nm, preferably at least 50 mW/nm.

33. Method of power treatment or analysis of tissue or biological samples with light, said light having mean wavelengths longer than 1100 nm, said light having a spectral power density of at least 20 mW/nm, preferably at least 50 mW/nm.
